# EUROPEAN PATENT APPLICATION

(11) **EP 3 825 004 A1**
(43) Date of publication of application: **26.05.2021**
(21) Application number: 19210858.7
(22) Date of filing: 22.11.2019
(51) Int. Cl.: B01L 3/00, A61B 10/00, G01N 1/36

(54) **NEW MULTI-FUNCTIONAL FLUIDIC DEVICE FOR CLAMPING BIOPSIES**

(71) Applicant: Koninklijke Philips N.V., 5656 AG Eindhoven (NL)
(72) Inventor: Beckers, Lucas Johannes Anna Maria, 5656 AE Eindhoven (NL)
(74) Representative: Philips Intellectual Property & Standards

(57) **Abstract**

A fluidic device (1) comprises a flow chamber (2) for accommodating a biological specimen on a carrier portion (3) and at least one flow channel (4a, 4b, 4c, 4d) fluidly connected to the flow chamber (2), the fluidic device (1) having a layered structure comprising a bottom plate (5), a cover plate (6) and an insert (7) in between, the insert (7) comprising the carrier portion (3) and a frame portion surrounding the carrier portion (3), and being elastomeric in order to be able to clamp a biological specimen between an incision in the carrier portion (3).

## Description

### FIELD OF THE INVENTION

The present invention relates to devices for holding a biological specimen in a fixed orientation and/or for supplying fluids to a biological specimen. The invention further relates to an insert for such devices, as well as a kit including such insert and at least one cover plate which together with the insert may provide a device of the invention. The present invention further relates to a system comprising the insert or the device of the invention. The present invention further relates to a use of the insert, kit or device for holding a biological specimen in a fixed orientation and /or supplying fluids to a biological specimen.

### BACKGROUND OF THE INVENTION

Prostate biopsy is a procedure in which a small tissue sample (biopsy) from a prostate gland of a patient is removed to be examined for the presence of prostate cancer. Most often, a cylindrical sample having an approximate diameter of 1 mm and length of e.g. between 5 and 20 mm is isolated using hollow needles, and the isolated sample. The sample usually is H&E stained for histological examination under a microscope to determine whether cancer cells are present and to evaluate microscopic features and/or Gleason score of any cancer found. From the results a clinical risk may be determined, which may then be used to indicate suitable treatments.

To prepare a tissue sample for histological examination, it must be prevented from decay due to autolysis or putrefaction. This is done by fixation with which ongoing biochemical reactions are terminated and mechanical strength or stability of the tissue sample may be increased. The tissue sample is then either embedded in paraffin or frozen to preserve tissue morphology and to give the tissue sample sufficient support during sectioning. The usual fixative for paraffin embedded tissues is neutral buffered formalin. Since in these processes the tissue sample must be brought in contact with many different fluids it is desirable to simplify supply of fluids to it.

While fluidic devices may foresee in the aforementioned desire, the use of such devices for preparation of tissue samples comes with difficulties. For instance, when tissue samples are placed on a surface of a flow chamber of such device, such surface generally impairs perfusion of fluids and reduces transparency needed for microscope investigation. When, on the other hand, the biopsy is allowed to freely move in the flow chamber, its orientation will be lost after some time and this may obstruct accurate investigation with regard to areas of most diagnostic value or 3D structure of the tissue sample.

There is thus a need for improved devices for use as tissue sample carrier in biopsy procedures in general.

### SUMMARY OF THE INVENTION

The invention aims to at least partly fulfil the aforementioned need.

In accordance with a first aspect of the invention, there is provided a fluidic device as claimed in claim 1 to address the aforementioned aim.

The fluidic device comprising a flow chamber for accommodating a biological specimen on a carrier portion and at least one flow channel fluidly connected to the flow chamber. The fluidic device has a layered structure comprising a bottom plate, a cover plate and an insert in between. This means that the insert is sandwiched between the bottom plate and the cover plate. Further, the insert comprises the carrier portion and a frame portion surrounding the portion. The carrier portion and preferably, but not necessarily is characterized by being elastomeric. Thus the carrier portion comprises or consist of an elastomer material. Optionally also the frame portion or the entire insert may comprise or consist of a further elastomer material. Preferably the elastomer material and the further elastomer material are the same.

The carrier portion may have the form of a membrane with a thickness much thinner than its width and length directions. The membrane may thus be stretchable, that is elastic.

The term "elastomeric" as used herein is meant to denote an object's mechanical properties that renders the object capable of stretching so as to return to an original shape and/or size when stretching force is released. The mechanical properties are in particular such that the object displays rubber-like elasticity in accordance with IUPAC's definition of an "elastomer" which is to mean an elastomer material. Accordingly, the phrases "elastomeric insert", "insert being elastomeric" and synonymous expressions characterize mechanical properties that renders the insert capable of stretching so as to return to an original shape and/or size when stretching force is released (i.e. reversibly stretchable). Further, an insert shall be understood to be elastomeric when the insert is reversibly stretchable in at least a region of the insert so that it becomes possible to clamp a biological specimen as described further below. This region may lie in or be constituted by the carrier portion and/or be constituted by a region adjacent to the carrier portion extending in opposite directions from the boundary around the carrier portion through the frame portion. However, it would be desirable that both uniform perfusion is enabled so that supplied fluids reach the whole surface of a biopsy and the orientation of a biopsy is maintained. Therefore the fluidic channels can be used. A biopsy has generally an area of most diagnostic value. Ideally, the specimen would be oriented so that all diagnostically valuable layers would be on a microtome slice for evaluation of the pathologist. Furthermore, to ease handling and the number of manual steps it would further be desirable that a sample can be analyzed directly in the fluidic device.

As mentioned above, the insert is intended to a clamp a biological specimen. As used herein, the term "biological specimen" is intended to mean one or more tissue, organism or portion thereof. A biological specimen can be obtained from any of a variety of organisms. Exemplary organisms include, but are not limited to, mammals such as a rodent, mouse, rat, rabbit, guinea pig, ungulate, horse, sheep, pig, goat, cow, cat, dog, primate (i.e. human or non-human primate). Preferably, the biological specimen is a biopsy. To clamp the biological specimen, the insert is elastomeric so that it can be reversibly stretched (pushed, pulled and/or bended). Further, the insert is modifiable so that it can be advantageously used to pick up, clamp and/or fix in place a biological specimen. This can be done by various approaches, some of which are exemplarily explained in the following.

Common to these approaches, an incision is provided in the portion, preferably as a cut through the whole layer thickness (full cut-through) of the microporous carrier portion, and the biological specimen is put between the incision by applying an external force to the insert. An incision as used herein denotes a slit-shaped opening that, when no external force is applied to the insert, leaves a gap between opposing cutting ends of less than 1000 µm, preferably less than 500 µm, more preferably less than 250 µm and most preferably less than 100 µm. The incision has preferably the shape of a straight line or a cross. By applying an external force to the insert the gap is widened so as to be able to receive a biological specimen. The external force may be applied as a bending force, a pulling force or a pushing force. Due to the elastomeric properties, the incised insert is able of clamping and/or fix in place a biological specimen, when no external force is applied to the insert.

According to one approach, the biological specimen is put between the incision while a pushing force is applied to the carrier portion directly onto or in immediate proximity of the incision in a direction of the layer thickness (i.e. from top or bottom, relative to the insert's orientation during use). The opening may, for instance, be widened with help of tweezers, which may at the same time serve to place the biological specimen between the incision. When the pushing force is ceased, the insert takes its original shape and the opening closes. This approach has the advantage that insertion of a biological specimen can be automatized and parallelized using existing fluidic device holders such as those disclosed herein.

According to another approach, the biological specimen is put between the incision while opposing edges of the insert are pulled away from each other so that the opening in the carrier portion is forced to widen. By ceasing the pulling force, the biological specimen is clamped between the incision. According to yet another approach, the insert is bent along an axis extending through the carrier portion so that opposing edges of the insert approach each other along a circular trajectory, the cut ends spread apart leaving a widened, wedge-shaped opening. Once the bending force is ceased, the insert takes its original shape and the opening closes. Thereby, a forceps-like clamping mechanism is created, which can not only be used to clamp and/or fix in place a biological specimen in a corresponding manner as above but may also serve to pick up the biological specimen.

When the biological specimen is clamped tight, the exact orientation of the biological specimen can be retained, chemicals and nutrition have free access to the biological specimen from top and bottom, and free optical view for microscopy can be provided.

Preferably the carrier portion comprises pores or microporous such that fluid can pass through the carrier portion.

The fluidic device as disclosed herein is in particular useful for performing experiments on a biopsy such as a prostate biopsy. As described in the introduction, a biopsy has an area of most diagnostic value, and, ideally, the specimen would be oriented so that all diagnostically valuable layers would be on a microtome slice for evaluation. While orientation is not achievable when a biological specimen such as a prostate needle biopsy is received in prefilled formalin containers, orientation can be retained through immobilization (i.e. clamping) by the insert forming part of the fluidic device as disclosed herein, while at the same time perfusion is enabled. This may be even improved by providing the carrier portion with the pores and preferably micropores. In this way perfusion may occur also occur through the pores or micropores. Hence, the biopsy may be treated with a plurality of different fluids containing for example nutrition, chemotherapeutic agents, staining agents and agents for preparing a biopsy such as formalin and paraffin within the same fluidic device. This fluidic device can be further used as a holder for a microtome to make slices for digital pathology and/or preparing 3D images of the biopsy. As further advantages, the fluidic device as disclosed herein has a simple design, can be easily manufactured and is cheap.

The fluidic device, as disclosed herein, is preferably a microfluidic device. A microfluidic device is understood to deal with manipulation of fluids that are geometrically constrained to a small, typically sub-centimeter or sub-millimeter, scale at which capillary penetration may govern mass transport, although this is not necessary.

The carrier portion comprises or consists of an elastomer material and/or the frame portion comprises or consists of a further elastomer material. An elastomer as understood herein is a polymer that displays rubber-like elasticity, as defined by IUPAC. The elastomer provides elasticity for reversibly stretching (i.e. pulling, pushing or bending) the insert as disclosed herein. The elastomer may be also based on more than one polymer, and each of the one or more polymers may be independently selected from homo-polymers, copolymer and polymer blends.

Preferred elastomers are based on at least one polymer selected from the group consisting of thermoplastic elastomers (TPE) and rubbers like silicones and polybutadiene rubbers, whereof particular preferred elastomers have been described in more detail in PCT application WO 2019/015988, which is herein incorporated by reference in its entirety, in particular for the purpose of disclosing elastomers for forming an insert (referred to as membrane in WO 2019/015988) of a fluidic device. The phrase "based on at least one polymer" as used herein describes an elastomer, wherein the at least one polymer is comprised as main constituent. In some embodiments, the at least one polymer makes up at least 60, at least 70, at least 80, at least 85, at least 90, at least 95 or at least 98 % by weight relative to the total weight of all constituents comprised in the microporous carrier portion. In some embodiments, the frame portion and/or the microporous carrier portion substantially consists or entirely consists of the at least one polymer.

Preferred thermoplastic elastomers are selected from the group consisting of styrenic block copolymers (TPS), preferably styrene ethylene butylene styrene block copolymers (SEBS), and thermoplastic polyurethanes (TPU). Particular preferred elastomers are characterized by an elasticity and/or Shore A hardness as disclosed herein. In preferred embodiments, the frame portion and/or the microporous carrier portion is made from an elastomer as disclosed herein. To simplify the design and production of the fluidic device of the present invention, the complete insert of particular preferred fluidic devices is made from an elastomer as disclosed herein.

In a similar vein, it is preferred that the microporous carrier portion is made from the same material as the frame portion and even more preferably the complete insert is made from the same material. In particular preferred fluidic devices, the insert is a one-part piece. This inter alia means that the microporous carrier portion and the frame portion are an integral part of the insert and, for instance, not glued together.

In a further preferred embodiment, the microporous carrier portion and/or the frame portion is characterized by a Shore A hardness of 80 or less and/or at least 2 (corresponding to a Shore OO hardness of 20). The Shore A hardness as disclosed herein define the hardness of certain portions of the insert. Generally, the Shore A hardness can be determined on a standard specimen that is of the same material as the respective portion to be assessed. There are different standard methods available, DIN ISO 7619, DIN EN ISO 868, ASTM.D 2240 or JIS K 6253-3. For the purposes of the present invention, the Shore A hardness is preferably 80 or less and/or at least 2, as determined in accordance to DIN EN ISO 868. If an elastomer comprises, in addition to one polymer, a further polymer and/or other constituents, the Shore A hardness as disclosed herein is meant to refer to the respective hardness resulting from the combination of all constituents present in the respective portion.

According to this embodiment, the insert's frame portion may have a hardness adapted to seal the structured region defining the flow paths as disclosed herein against fluid leakage. Due to use of a frame portion having a hardness as disclosed herein no additional sealing components such as gaskets are required. Instead, tight sealing can be obtained by compressing the layered structure, for example by placing the layered structure into a fluidic device holder as disclosed herein. With respect to the microporous carrier portion, a material having a hardness as disclosed herein is easily stretchable and provides sufficient elasticity for tightly clamping a biological specimen. In terms of simplicity of design and ease of manufacturing it is further preferred that the complete insert is characterized by a Shore A hardness of 80 or less and/or at least 2.

In a further preferred embodiment, the microporous carrier portion is provided with an incision. As described above, the incision is intended to clamp a biological specimen. The incision may be pre-formed or may be made, for instance by cutting with a knife, scissors, a razor blade or the like, before use. The microporous carrier portion may also have a designated region for making an incision, which may be optionally provided with a marking and/or a preformed breaking line, for instance a notch, along which line the microporous carrier portion needs to be cut through or breaks upon stretching. An incision or a pre-formed breaking line, as disclosed herein, may have a length of 0.3 to 30 mm, preferably 0.4 to 20 mm and more preferably 0.5 to 15 mm. It does preferably not extend into and does not extend through the frame portion.

In a further preferred embodiment, the flow chamber and the at least one flow channel are formed by structuring a lower surface and/or on an upper surface of the insert. In this case, the bottom plate and the cover plate may have a substantially flat shape and can be easily produced.

In a further preferred embodiment, the microporous carrier portion separates the flow chamber in a lower flow chamber region and an upper flow chamber region. Preferably, at least one lower flow channel is preferably fluidly connected to the lower flow chamber region and at least one upper flow channel is fluidly connected to the upper flow chamber region. According to such configuration, the lower flow chamber region and the upper flow chamber region can be fed with different fluids at the same time. For instance, a first fluid containing nutrients can be supplied to the lower flow chamber region, while a second fluid containing staining agents is supplied to the upper flow chamber region, or vice versa.

In a further preferred embodiment, the bottom plate and/or the cover plate comprises at least one port fluidly connected to the at least one flow channel. Supply of fluids through ports comprised in the cover plate can be easily implemented and readily used with existing instruments (e.g. fluid device holders).

To facilitate optical analyses, at least one of the bottom plate and the cover plate is preferably UV-VIS transparent. For this purpose, the bottom plate and/or the cover plate is preferably made of glass such as borosilicate glass. In addition to optical transparency, glass has the advantage of being hydrophilic, chemically inert, stable and nonporous. However, one will appreciate that, depending on the wavelength intended to be used for analysis, it is also possible to utilize UV-VIS transparent plastics such as PMMA or PS.

In accordance with a second aspect of the present invention, the above problem is solved by an insert as disclosed herein. The insert in particular comprises a microporous carrier portion and a frame portion surrounding the microporous carrier portion, the insert being elastomeric as described herein. Preferably, the frame portion and preferably also the microporous carrier portion is characterized by a Shore A hardness as disclosed herein. As described above, the insert may contain a plurality of microporous carrier portions, each microporous carrier portion being surrounded by the frame portion. The plurality of microporous carrier portions may be arranged in a rectangular pattern comprising, for instance, 4 to 20 by 6 to 40 carrier portions. The microporous carrier portions are preferably formed by depressions in the upper and/or lower surface of the insert. It is further preferred that at least one groove extends from each microporous carrier portion in order to provide at least one channel fluidly connected to each flow chamber, as disclosed herein.

In accordance with a third aspect of the present invention, the above problem is solved by a kit comprising an insert as disclosed herein, a cover plate as disclosed herein and a bottom plate as disclosed herein. It is to be understood that the bottom plate, the insert and the cover plate are such that, when stacked in this order, form a fluidic device as disclosed herein.

In accordance with a fourth aspect of the present invention, the above problem is solved by a system comprising a fluidic device holder and a fluidic device as disclosed herein, an insert as disclosed herein or a kit as disclosed herein. The fluidic device holder is understood to be adapted for engaging the fluidic device. For this purpose, the fluidic device holder may comprise a base plate on which a first outer surface of the fluidic device (in particular the lower surface of the bottom plate as disclosed herein) comes to lie and a compression plate adapted to exert a compression force on a second outer surface lying opposite of the first outer surface of the fluidic device (in particular the upper surface of the top plate as disclosed herein). The compression plate is intended to provide a compression force so that the fluidic device is clamped between the base plate and the compression plate of the fluidic device holder. Suitable device holders are commercially available, for instance, from Micronit B.V, Enschede, or Fluigent, Le Kremlin Bicêtre, and disclosed, for instance, in PCT application WO 2017/096296 A1 or WO 2009/002152 A1.

In accordance with a fifth aspect of the present invention, the above problem is solved by a method for clamping a biological specimen, preferably a biopsy such as a prostate biopsy, comprising the steps of providing an insert as disclosed herein, wherein the microporous carrier portion of the insert is provided with an incision, and clamping the biological specimen between adjacent cut ends defined by the incision. As explained with respect to the function of the fluidic device disclosed herein, clamping the biological specimen between adjacent cut ends defined by the incision can be affected by exerting a stretching force (i.e. pushing force, pulling force or bending force) as disclosed herein so that an opening is formed between adjacent cut ends defined by the incision, placing the biological specimen into the opening and ceasing the force, thereby clamping the biological specimen between the adjacent cut ends. Preferably, the biological specimen is clamped so that the exact orientation of the biological specimen can be retained, chemicals and nutrition have free access to the biological specimen from top and bottom, and/or free optical view for microscopy can be provided.

Preferred are also methods as disclosed herein further comprising sandwiching the insert between a top plate and a cover plate to form a fluidic device as disclosed herein.

Also preferred are methods as disclosed herein further comprising supplying at least one fluid to the flow chamber of the fluidic device. The fluid may comprise at least one agent selected from the group consisting of nutrients, chemotherapeutic agents, staining agents and agents for preparing a biopsy, in particular embedding and fixing agents such as paraffin and formalin.

According to particular preferred methods as disclosed herein, the biological specimen is pathologically and/or histologically examined, for instance visually by eyes or by microscopy. Prior to examination, the cover plate, in further preferred methods, is removed and the biological specimen, while still being clamped in the insert, is cut in slices, e.g., using a microtome. In such method, the insert is used as a holder for a microtome.

It shall be understood that the fluidic device, the insert, the kit, the system and the method as disclosed herein and in particular as disclosed in the appended independent claims have similar and/or identical preferred embodiments, in particular, as defined in the dependent claims.

It shall be understood that a preferred embodiment of the present invention can also be any combination of the dependent claims or the various embodiments described herein with the respective independent claim.

These and other aspects of the invention will be apparent from and elucidated with reference to the embodiments described herein.

### BRIEF DESCRIPTION OF THE DRAWINGS

Fig. 1 shows an example of a fluidic device as disclosed herein;
Fig. 2 shows a further example of a fluidic device as disclosed herein;
Figs. 3 and 4 respectivly show exploded bottom and top view of the fluidic device of Fig. 1;
Fig. 5 shows another example of a fluidic device as disclosed herein;
Fig. 6 shows yet another example of a fluidic device as disclosed herein;
Fig. 7 shows a sectional view of the fluidic device shown in Fig.1;
Figs. 8A and 8B show a top views of a part of a flow chambers having microporous carrier portions;
Fig. 9 shows an insert comprising a plurality of carrier portions;
Fig. 10 shows force-strain measurement results obtained by pushing a pen with rounded tip and a diameter of 1.2 mm in the thickness direction onto and through an opening in a membrane incision that was pre-cut in an insert, wherein Fig. 10A illustrates the measurement by showing pen positions at different time points, and wherein Fig. 10B is a graph of force (F) versus strain (Δ1); and
Fig. 11 shows results on force-strain measurements for a case where an insert having an incision as disclosed herein is stretched by pulling opposite ends of the insert away from each other in opposite directions, wherein Fig. 11A illustrates the measurement by showing the resulting opening at different time points during the measurement, and wherein Fig. 11B is a graph depicting force (F) versus strain (Δ1).

### DETAILED DESCRIPTION OF THE EMBODIMENTS

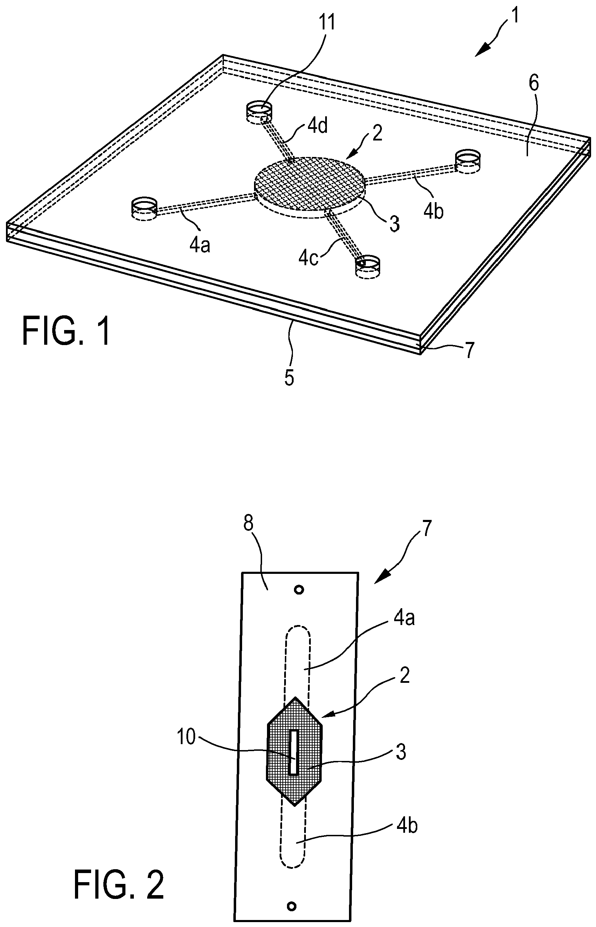

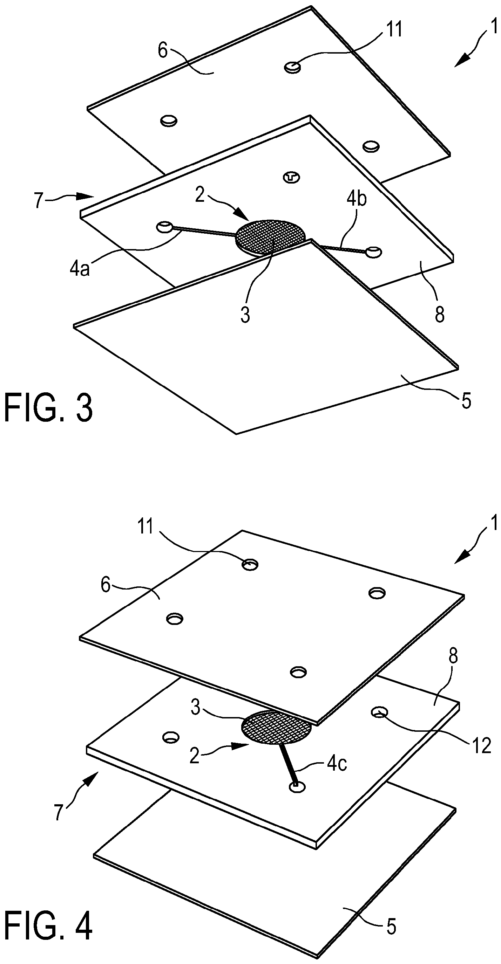

Referring to Fig. 1, a fluidic device 1 as disclosed herein comprises a flow chamber 2 for accommodating a biological specimen on a microporous carrier portion 3 and at least one flow channel 4, 4a, 4b, 4c, 4d fluidly connected to the flow chamber 2. The fluidic device 1 has a layered structure comprising a bottom plate 5, a cover plate 6 and an insert 7 in between, as can be best seen from the exploded views of Figs. 3 and 4. Further, the insert 7 comprises a microporous carrier portion 3 and a frame portion 8 surrounding the microporous carrier portion 3 and is modified or modifiable with an incision 9 provided in the microporous carrier portion 3, as can be seen in Fig. 2. In accordance with the present invention, the insert 7 is elastomeric so as to provide a clamping mechanism for a biological specimen as described herein.

To prevent damage of a biological specimen, the insert 7 is adapted so that the opening defined by the incision 9 can be widened to an extent sufficient to place the biological specimen in between using low force. This is particularly useful when handling a biopsy which is generally very weak and sensitive to mechanical stress. The extent of widening and the required force depends on the way how the incision 9 is forced to be opened.

In a case where opposing edges of the insert 7 are pulled in opposite directions away from each other, the force is preferably 5 N or less, more preferably 2.5 N or less and most preferably 1 N or less and/or at least 0.4 N, preferably at least 0.6 N. These values in particular apply when the width of the opening defined by the incision 9 is widened to 0.2 to 3 mm, preferably 0.4 to 2 mm, more preferably 0.5 to 1.5 mm and most preferably 1 mm ± 0.2 mm.

In a case where a pushing force is exerted in the thickness direction of the insert 7 directly onto the incision 9 or in immediate proximity thereto using, e.g., tweezers, the force is preferably 50 mN or less, more preferably 10 mN or less and most preferably 5 mN or less and/or at least 0.5 mN, preferably at least 1 mN.

In a case where a bending force is exerted along an axis extending through the microporous carrier portion 3 so that the insert 7 bends by at least 60°, preferably at least 90°, more preferably at least 120°, even more preferably at least 150° and most preferably about 180°C, the force when grasping opposing edges of the insert 7 by a thumb and a forefinger is preferably 1000 mN or less, more preferably 500 mN or less and most preferably 200 mN or less and/or at least 2 mN, preferably at least 5 mN.The flow chamber 2, as disclosed herein, denotes a (micro-) structured region within the fluidic device 1, which has a volume sufficient to accommodate a biological specimen 10, preferably a biopsy such as a prostate biopsy, and at least one inlet and/or at least one outlet connecting to the at least one channel 4a, 4b, 4c, 4d. Preferred flow chambers 2 have a volume of 50 to 1000 mm³, for example 100 to 500 mm³. In specific embodiments, the flow chamber 2 further provides a microenvironment that enables a biological specimen 10 to be cultivated and/or to retain functional activity. In this case, the microporous carrier portion 3 may be provided with a correspondingly functionalized surface. For instance, the microporous carrier portion 3 may be functionalized with collagen gel or fibronectin. In this way, cells or tissue may be accommodated in an environment similar to their naturally occurring environment, which is particularly useful when the fluidic device is used for cultivating the biological specimen (cells or tissue) for organ replacement / transplantation (organ-on-a-chip), performing ex-vivo chemotherapy on the biological specimen or testing pharmaceuticals on the biological specimen. Suitable functionalization agents and methods are disclosed, for instance, in PCT application WO2019/015988, titled "Cell culturing materials", which disclosure is herein incorporated by reference in its entirety, in particular for the purpose of disclosing agents and methods for functionalizing an insert for a fluidic device.

According to the shown embodiment, the flow chamber 2, when viewed from the top, has an essentially circular form. More precisely, the flow chamber 2 preferably has a shape of a flat cylinder. The flow chamber 2 can have a diameter of, for example, 2 to 30 mm, preferably 3 to 20 mm and more preferably 4 to 15 mm. Other shapes are also envisaged such as ellipsoid or rectangular forms.

A microporous carrier portion 3 and a frame portion 8, as disclosed herein, can be best seen in Fig. 2. Here, it can also be seen that the at least one channel 4a, 4b (an inflow channel and an outflow channel in the embodiment of Fig. 2) branches off from the flow chamber 2 and extends into the frame portion 8. Preferably, the bottom plate 5 and the cover plate 6 are relatively stiff, whereas the insert 7 is relatively soft, as will be further explained herein. With continuing reference to Fig. 2, it can be seen that an incision 9 is preferably provided in the microporous carrier portion 3.

The term "one channel" as used herein denotes a configuration that provides s single channel for inflow and outflow of fluids and a configuration that provides a pair of channels, one of which is provided for inflow of fluids (inflow channel) and the other one being provided for outflow of fluids (outflow channel). A configuration comprising such pair of inflow channel 4a and outflow channel 4b can be seen in Fig. 3. A configuration comprising two pairs of channels, each pair comprising an inflow channel 4a, 4c and a separate outflow channel 4b, 4d is best seen in Fig. 1. Fluidic devices 1 as disclosed herein may comprise 1 flow channel, 2 flow channels, 3 flow channels, 4 flow channels, 5 flow channels, 6 flow channels or even more than 6 flow channels as disclosed above.

Referring again to the exploded view of the fluidic device 1 shown in Fig. 3, it can be seen that the insert's frame portion 8 is preferably intended to define the chamber's side walls and space apart the bottom plate 5 and the cover plate 6. This means, the flow chamber 2 is enclosed by the upper surface of the bottom plate 5, the lower surface of the cover plate 6 and the inner side walls of the frame portion 8. In the shown embodiment, the frame portion 8 is constituted by the unstructured region of the insert 7. However, it can be readily appreciated that the frame portion 8 can be alternatively formed by an elevation that circumscribes the structured region defining the flow chamber 2 and the at least one flow channel 4 (herein collectively referred to as flow paths 2, 4).

As can be best seen in Figs. 3 and 4, the flow chamber 2 and the at least one flow channel 4a, 4b, 4c, 4d of fluidic devices 1 as disclosed herein are preferable formed by structuring the insert 7. However, it is also envisaged that at least one of the bottom plate 5 and the cover plate 6 is structured. In preferred fluidic devices 1, a lower surface of the insert 7 (i.e. surface facing the bottom plate 5) and an upper surface of the insert 7 (i.e. surface facing the cover plate 6) is provided with appropriate structures forming the flow paths 2, 4 as disclosed herein. More specifically, the lower surface of the insert 7 is preferably provided with at least one groove in a region of the frame portion 8 to form the at least one channel 4a, 4b, as shown in Fig. 3. In addition or alternatively, the upper surface of the insert 7 may be preferably provided with at least one groove in a region of the frame portion 8 to form the at least one channel 4c, 4d, as shown in Fig. 4. The cross-sectional shape of the at least one groove corresponding to the at least one channel 4 is not limited, and examples of possible shapes include recessed shapes, U-shapes, and V-shapes. The depth and/or width of the groove is preferably 10 to 2000 µm, more preferably 50 to 1500 µm and most preferably 100 to 1000 µm.

It is further preferred that a depression is provided on the lower surface (cf. Fig. 3) and/or on the upper surface (cf. Fig. 4) of the insert 7 in a region of the carrier portion 3 to form the flow chamber 2. According to this configuration, the flow chamber 2 is formed by a microporous carrier portion 3 having a lower layer thickness than the layer thickness of the surrounding frame portion 8. Such preferred inserts 7 can be produced by any suitable technique for producing a surface-structured - preferably elastomeric - article. Preferred techniques include, without being limited, molding, 3-D printing, casting and embossing.

With continuing reference to Figs. 3 and 4, it can be seen that fluids are preferably supplied through the cover plate 6. For this purpose, the cover plate 6 comprises at least one port 11 that provides fluid access to the at least one flow channel 4a, 4b, 4c, 4d. It can be further seen that the insert 7 may comprise at least one aperture 12 aligning with the at least one port 11, the at least one aperture 12 being fluidly connected to the flow chamber 2 via the at least one flow channel 4. In the embodiment shown in Figs. 3 and 4, the at least one port 11 and the at least one aperture 12 have a circular shape with coinciding radial axes. Although supply of fluids through the cover plate 6 as disclosed above can be easily implemented and readily used with existing instruments (e.g. fluid device holders), it is also envisaged that fluids can be supplied through the bottom plate 5 or through a side surface of the insert 7, in which case corresponding port(s) 11 is/are present in the bottom plate 5 or the insert 7.

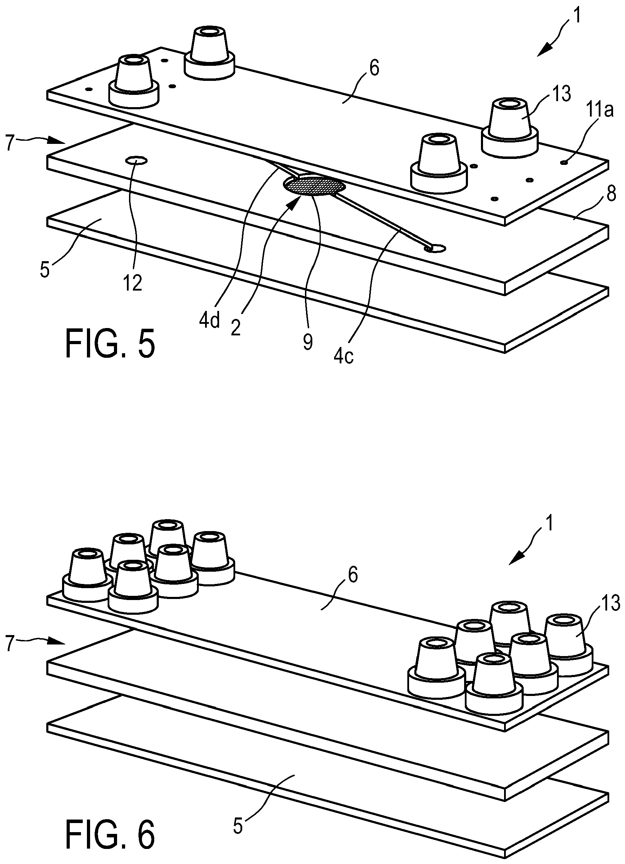

As shown in Figs. 5 and 6, the port 11 may be connected to a fitting 13, which is connectable to a tube carrying fluid. The embodiment shown in Fig. 5 has a configuration with 4 fittings 13 providing fluid access to a lower flow channel (not seen in Fig. 5) and an upper flow channel 4c, 4d, as disclosed herein. It can be further seen that the cover plate 6 is provided with a plurality of ports 11, whereas the number of functional ports (i.e. ports providing fluid access to the flow chamber 2 via the at least one flow channel 4a, 4b, 4c, 4d) is governed by the structured surface of the insert 7. In this example, the fluidic device 1 comprises 4 functional ports and 8 dead-end ports 11a, which do not align with a corresponding aperture 12 of the insert 7 and are therefore not functional. In the configuration shown in Fig. 6, all 12 ports are provided with fittings 13 and are functional. The structuring of the insert 7 is not shown. This embodiment has the advantage in that an equally configured cover plate 6 can be used irrespective of the desired number of ports being functional.

The insert 7 is preferably a flat cuboid with an upper surface and a lower surface facing a lower surface of the cover plate 6 and an upper surface of the bottom plate 5, respectively. In the upper surface of the flat cuboid and/or lower surface of the flat cuboid structured regions are provided that form flow paths 2, 4 as disclosed herein. It is further preferred that the upper and the lower surfaces of the insert 7 are rectangular with a longer and a shorter edge length, and that the incision is provided substantially parallel (± 45°, preferably ± 30° and more preferably ± 20°) to the shorter edge length. Other shapes, such as a flat cuboid shape with rounded edges or a flat cylindrical shape, with corresponding structures as described above are conceivable as well. It is further preferred that the shape of the insert 7 is such that its upper and lower surfaces correspond to the lower surface of the cover plate 6 and the upper surface of the bottom plate 5, respectively.

The layer thickness of the - preferably flat cuboid - insert 7 can, when measured at the thickest point, range between 0.5 and 20 mm, and is preferably 1 to 15 mm, more preferably 2 to 10 mm and most preferably 3 to 8 mm. The layer thickness of the insert 7 can, when measured at the thinnest point, range between 0.1 and 5 mm, and is preferably 0.1 to 3 mm, more preferably 0.2 to 2 mm and most preferably 0.3 to 1 mm. In preferred fluidic devices 1, the thickest point lies in the frame portion 8 and the thinnest point lies in the carrier portion 3. In this case, the carrier portion 3 may have, for example, a thickness that is 5 to 50 % of the thickness of the frame portion 8.

A preferred fluidic device 1 as disclosed herein comprises a planar bottom plate 5 and/or a planar cover plate 6, wherein the planar bottom plate 5 and/or the planar cover plate 6 has a flat cuboid shape with optionally rounded edges or a flat cylindrical shape as described above with respect to the insert 7. The thickness of the - preferably flat cuboid - bottom plate 5 and/or cover plate 6 can be in a range of 0.5 and 20 mm, and is preferably 0.8 to 15 mm, more preferably 1 to 10 mm and most preferably 2 to 6 mm.

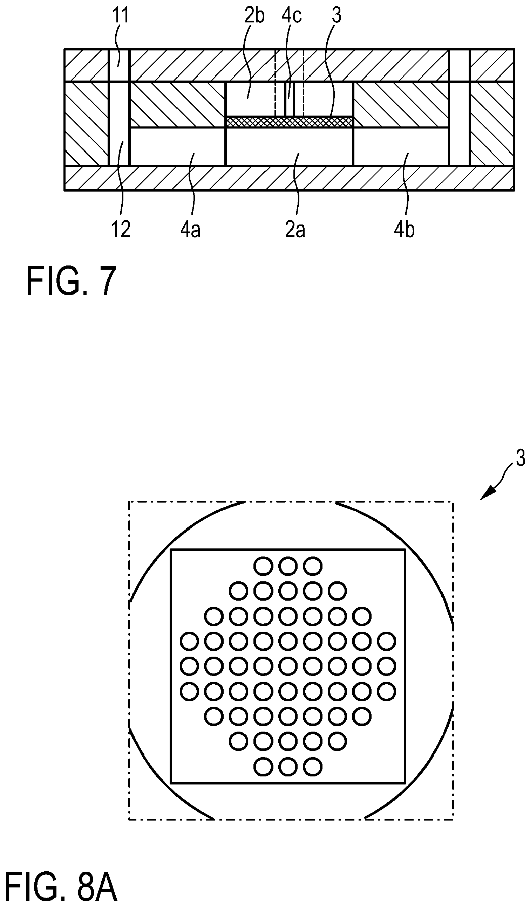

Referring now to Fig. 7, a sectional view of the fluidic device 1 shown in Figs. 3 and 4 is shown. As can be seen, in preferred fluidic devices 1, the carrier portion 3 separates the flow chamber 2 in a lower flow chamber region 2a and an upper flow chamber region 2b. In this case, it is further preferred that at least one lower flow channel 4a, 4b is provided that stands in fluid connection with the lower flow chamber region 2a and at least one upper flow channel 4c, 4d is provided that stands in fluid connection with the upper flow chamber region 2b. According to such configuration, the lower flow chamber region 2a and the upper flow chamber region 2b can be fed with different fluids at the same time as described herein. As also described herein, the at least one channel 4 is preferably a pair of channels, which pair provides an inflow channel 4a, 4c and an outflow channel 4b, 4d.

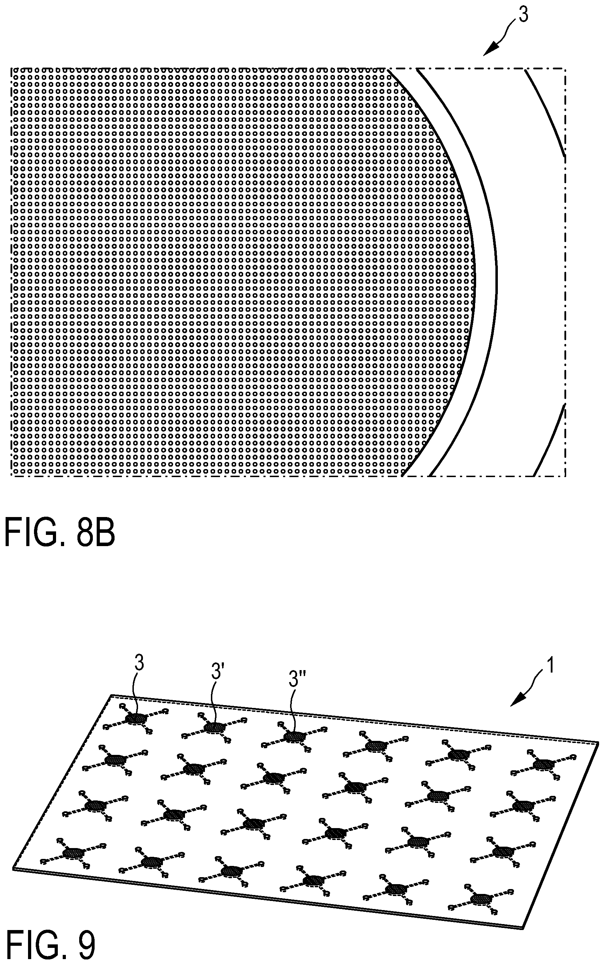

Next, the structure of the microporous carrier portion 3 of preferred fluidic devices 1 is described with reference to Fig. 8. The structure is especially such that cross-sectional fluid flow through the insert can occur and thereby constant and reproducible perfusion can be enabled, and that the insert 7 can be reversibly bent along an axis extending through the microporous carrier portion 3. Number and size of pores as well as pore density can be selected according to needs. Further, the pores may be randomly distributed or arranged in a geometrically ordered pattern. In the microporous carrier portion 3 shown in Fig. 8A, the pores are arranged in a geometrically ordered and symmetric pattern. Such pattern may be polygonal, for instance octagonal as shown, or have any other form including rectangular, circular or ellipsoid forms. According to an exemplary embodiment, a microporous carrier portion 3 as disclosed herein may comprise a multitude of pores, for example 8 to 200 pores, 12 to 150 pores, 16 to 100 pores or 20 to 80 pores. In this case, the pore mean diameter may preferably be relatively large, for instance, 100 µm to 1000 µm, 200 µm to 750 µm or 250 µm to 500 µm. The pore density may be 8 to 200 pores/cm², 12 to 150 pores/cm², 16 to 100 pores/cm² or 20 to 80 20 pores/cm². As illustrated in Fig. 8B, the microporous carrier portion 3, according to another exemplary embodiment, comprises 20 to 1500 pores, 50 to 1000 pores or 100 to 750 pores. In this case, the pore mean diameter is preferably relatively small, for instance, 5 µm to 100 µm, 8 µm to 75 µm or 10 µm to 50 µm. The pore density may be 20 to 1500 pores/cm², 50 to 1000 pores/cm² or 100 to 750 pores/cm². The pores can be conveniently produced by molding using appropriate matrices, or forming a mesh using conventional weaving techniques.

Referring to Fig. 9 it can be seen that fluidic devices 1 as disclosed herein are not limited insofar as they comprise only one flow chamber 2. Fluidic devices 1 as disclosed herein may rather comprise more than one flow chamber 2. For example, there may be a plurality of flow chambers, and a plurality of corresponding carrier portions 3, 3', 3" may be comprised in the insert 7 sandwiched between a bottom plate 5 and a cover plate 6 as disclosed herein. In the embodiment shown in Fig. 9, the flow chambers 2 are arranged in a pattern comprising 6 columns by 4 rows. A single bottom plate 5 and a single cover plate 6 extend over opposing surfaces of the insert 7 and thus seal the plurality of flow chambers 2a, 2b, 2c and flow channels 4. The cover plate 6 further comprises a plurality of ports 11 providing fluid access to the plurality of flow chambers 2a, 2b, 2c via flow channels 4.

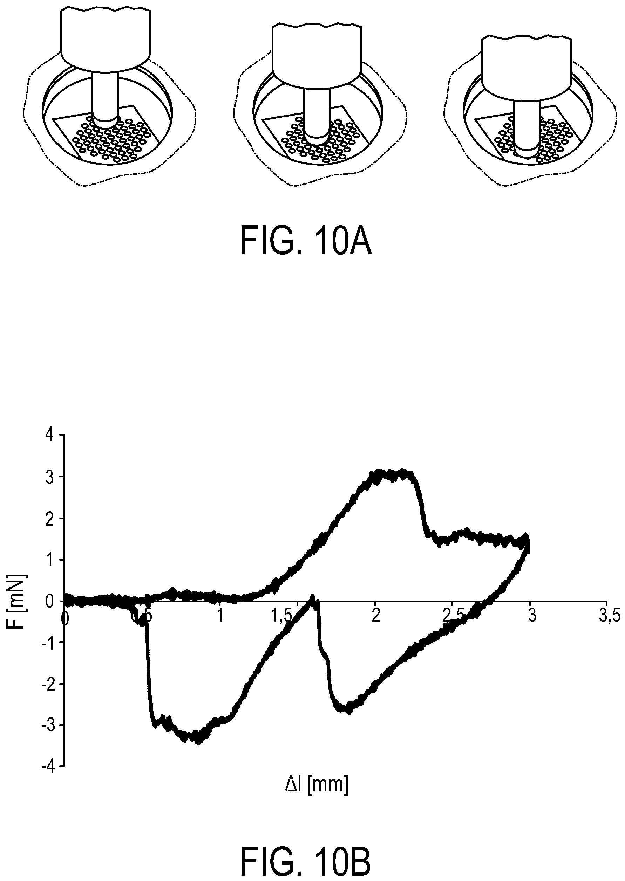

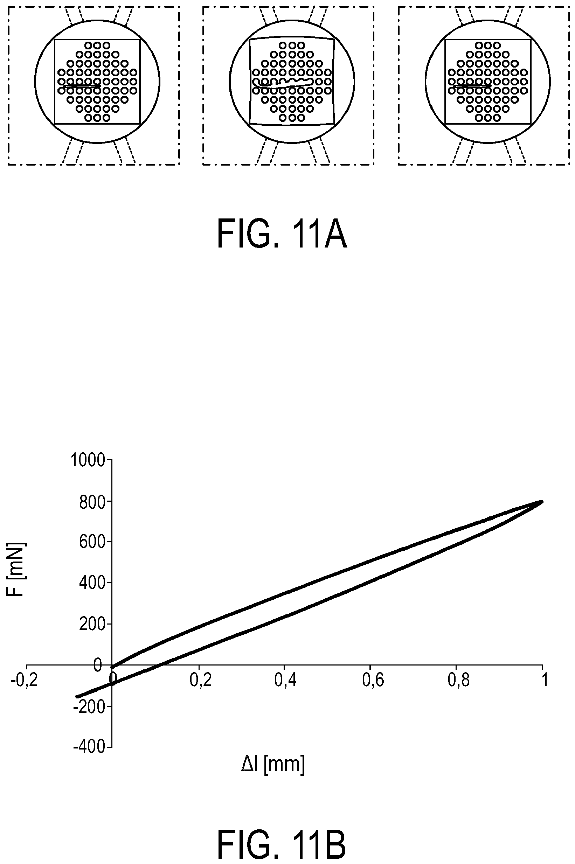

Referring to Figs. 10 and 11, results on stress-strain measurements on inserts 7 as disclosed herein are shown. Fig. 10 are measurements where the forces needed to push tweezers with a biological specimen between the incision 9 and leave the biological specimen behind were simulated. The forces needed to manipulate the opening and closing of the insert 7 were simulated on a Zwick Z010 stress bench by pushing a pen with rounded tip and having a diameter of 1.2 mm in thickness layer direction onto and through an incision 9 that was pre-cut in an insert 7. Following three individual measurements, the forces were determined to be in the order of 3 mN.

Fig. 11 are measurements where the forces needed to widen an opening defined by the incision 9 by pulling to an extent so that the widened opening is able to receive a biological specimen, for instance to a width of 1 mm, were simulated. The forces needed to manipulate the opening of the insert 7 were simulated on a Zwick Z010 stress bench by pulling opposite ends of the insert 7 away from each other in opposite directions. The insert 7 was made of silicone having a Shore A hardness of 40 (Wacker Elastosil 3040/40). The microporous carrier portion 3 of the insert 7 was 35 µm thick and the pore diameter was 200 µm. Following an exemplary measurement, the results of which are shown in Fig. 11, the forces for widening the opening to a width of 1 mm were determined to be in the order of 800 mN.

Other variations to the disclosed embodiments can be understood and effected by those skilled in the art in practicing the claimed invention, from a study of the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A fluidic device (1) comprising a flow chamber (2) for accommodating a biological specimen (10) on a carrier portion (3) and at least one flow channel (4, 4a, 4b, 4c, 4d) fluidly connected to the flow chamber (2), the fluidic device (1) having a structure comprising a bottom plate (5), a cover plate (6) and an insert (7) in between the bottom plate and the cover plate, the insert (7) comprising the carrier portion (3) and a frame portion (8) surrounding the carrier portion (3), the carrier portion comprising or consisting of an elastomeric material.

2. The fluidic device (1) of claim 1, wherein the frame portion (8) comprises or consists of a further elastomeric material, wherein preferably the further elastomeric material is the same as the elastomeric material.

3. The fluidic device (1) of claim 1 or 2, wherein the carrier portion (3) and/or the frame portion (8) is **characterized by** a Shore A hardness within the range of 2 to 80.

4. The fluidic device (1) of any of the preceding claims, wherein the carrier portion (3) is provided with an incision (9) or a region for making an incision.

5. The fluidic device (1) of any of the preceding claims, wherein the elastomeric material and/or the further elastomeric material are chosen from the group consisting of thermoplastic elastomers and elastomers like silicones and polybutadiene rubbers, wherein the thermoplastic elastomers are preferably selected from the group consisting of styrenic block copolymers (TPS), preferably styrene ethylene butylene styrene block copolymers (SEBS), and thermoplastic polyurethanes (TPU).

6. The Fluidic device of any of the previous claims wherein the carrier portion is porous, or microporous.

7. The fluidic device (1) of any of the preceding claims, wherein the flow chamber (2) and the at least one flow channel (4, 4a, 4b, 4c, 4d) are formed by structuring a lower surface and/or on an upper surface of the insert (7).

8. The fluidic device (1) of any of the preceding claims, wherein the microporous carrier portion (3) separates the flow chamber (2) in a lower flow chamber region (2a) and an upper flow chamber region (2b), and
wherein at least one lower flow channel is preferably fluidly connected to the lower flow chamber region (2a) and at least one upper channel is fluidly connected to the upper flow chamber region (2b).

9. The fluidic device (1) of any of the preceding claims, wherein the bottom plate (5) and/or the cover plate (6) comprises at least one port (11) fluidly connected to the at least one flow channel (4, 4a, 4b, 4c, 4d).

10. The fluidic device (1) of any of the preceding claims, wherein the bottom plate (5) and/or the cover plate (6) is UV/VIS transparent.

11. The fluidic device (1) of any of the preceding claims, wherein the bottom plate (5) and/or the cover plate (6) has a layer thickness of 0.5 to 20 mm, preferably 0.8 to 15 mm, more preferably 1 to 10 mm and most preferably 2 to 6 mm, and/or
wherein the insert (7) has a layer thickness, when measured at the thickest point, of 0.5 and 20 mm, preferably 1 to 15 mm, more preferably 2 to 10 mm and most preferably 3 to 8 mm and/or
wherein the insert (7) has a layer thickness, when measured at the thinnest point, of 0.1 and 5 mm, preferably 0.1 to 3 mm, more preferably 0.2 to 2 mm and most preferably 0.3 to 1 mm.

12. An insert (7) for a microfluidic device (1), the insert (7) comprising a carrier portion (3) and a frame portion (8) surrounding the carrier portion (3), the insert (7) comprising or consisting of an elastomeric material, wherein the insert (7) is as defined as in any of the claims 2 to 6.

13. The insert (7) of claim 11, wherein the insert (7) contains a plurality of carrier portions (3a, 3b, 3c), each carrier portion (3a, 3b, 3c) being surrounded by the frame portion (8).

14. A system or kit comprising an insert (7), a bottom plate (5) and a cover plate (6), wherein the bottom plate (5), the insert (7) and the cover plate (6), when stacked in this order, form a fluidic device (1) as defined in any of claims 1 to 11.

15. The system or kit of claim 14 comprising:
- a fluidic device (1) as claimed in any of claims 1 to 11, or
- an insert (7) as claimed in claim 12 or 13; and
- a holder for holding the fluidic device.

16. A method for clamping a biological specimen (10), preferably a biopsy, comprising:
providing an insert (7) as defined in claim 11 or 12, wherein the microporous carrier portion (3) of the insert (7) is provided with an incision (9), and
clamping the biological specimen (10) between adjacent cut ends defined by the incision (9).
